(19) Europäisches Patentamt
European Patent Office
Office européen des brevets

(11) Veröffentlichungsnummer: **0 000 336**
**B1**

# (12) EUROPÄISCHE PATENTSCHRIFT

(45) Veröffentlichungstag der Patentschrift: **15.04.81**

(21) Anmeldenummer: **78100184.7**

(22) Anmeldetag: **19.06.78**

(51) Int. Cl.³: **C 07 D 239/48,**
A 61 K 31/505,
C 07 D 401/12,
C 07 D 403/12,
C 07 D 413/12

## (54) Amidino-benzylpyrimidine, Verfahren zu ihrer Herstellung und diese enthaltende Arzneimittel.

(30) Priorität: **15.07.77 DE 2732029**

(43) Veröffentlichungstag der Anmeldung:
**24.01.79 Patentblatt 79/2**

(45) Bekanntmachung des Hinweises auf die Patenterteilung:
**15.04.81 Patentblatt 81/15**

(84) Benannte Vertragsstaaten:
**BE CH DE FR GB LU NL SE**

(56) Entgegenhaltungen:
**Chemical Abstracts, Vol. 79 (1973), Nr. 19, Seite 362, Spalte 2, P 115.615c.**

(73) Patentinhaber: **BASF Aktiengesellschaft
Carl-Bosch-Strasse 38
D-6700 Ludwigshafen (DE)**

(72) Erfinder: **Scharwaechter, Peter, Dr.
An der Duene 9
D-2082 Moorrege (DE)**
Erfinder: **Gutsche, Klaus, Dr.
Ehmschenkamp 5
D-2084 Rellingen (DE)**
Erfinder: **Kohlmann, Wilhelm, Dr.
An der Duene 6
D-2082 Moorrege (DE)**
Erfinder: **Kroemer, Gerd, Dr.
verstorben, zuletzt wohnhaft in Kaltenweide 100a
D-2200 Elmshorn (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 000 336 B1**

# Amidino-benzylpyrimidine, Verfahren zu ihrer Herstellung und diese enthaltende Arzneimittel

Die Erfindung betrifft Amidino-benzylpyrimidine der allgemeinen Formel I

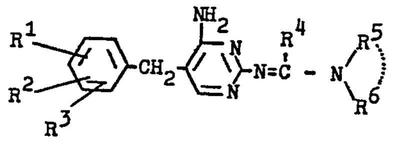

(I)

in der $R^1$, $R^2$ und $R^3$, die gleich oder verschieden voneinander sein können, Wasserstoff, Methyl, Methoxy oder Chlor bedeuten, $R^4$ Alkyl mit 1 bis 6 C-Atomen oder Benzyl bedeutet und $R^5$ und $R^6$, die gleich oder verschieden voneinander sein können, Wasserstoff, niederes Alkyl mit 1 bis 4 C-Atomen, Trimethoxybenzyl, Benzyl oder Phenyl, Cyclohexyl, Adamantyl oder Furfuryl oder einer der Reste $R^5$ oder $R^6$ die Gruppierung $—C_6H_4—SO_2—NH—R^7$ bedeutet, wobei $R^7$ den Rest

$$-\overset{\overset{\displaystyle NH}{\|}}{C}-NH_2, \qquad -\overset{\overset{\displaystyle O}{\|}}{C}-NH_2$$

oder den Pyrimidin-2-yl-, 4-Methyl-pyrimidin-2-yl, 5-Methyl-pyrimidin-2-yl, 5-Isopropyl-pyrimidin-2-yl,-5-Methoxy-pyrimidin-2-yl, 6-Methoxy-pyridazin-3-yl, 3-Methoxy-pyrazin-2-yl-, 5-Methyl-isoxazol-3-yl oder 2-Pyridinyl-Rest bedeutet, oder in der $R^5$ und $R^6$ gemeinsam mit dem Stickstoff, mit dem sie verbunden sind, einen gesättigten heterocyclischen Ring mit 5 bis 7 Gliedern bilden, der gegebenenfalls ein Sauerstoffatom oder die N—Y—Gruppe enthalten kann, wobei Y für Methyl, Benzyl oder Phenyl steht, sowie deren pharmakologisch verträglichen Salze mit hierfür üblichen Säuren.

Als übliche Säuren zur Bildung pharmakologisch verträglicher Salze kommen beispielsweise Salzsäure, Salpetersäure, Schwefelsäure, Phosphorsäure, Essigsäure, Milchsäure, Citronensäure und Salicylsäure in Frage.

Bevorzugt stehen die Substituenten $R^1$, $R^2$ und $R^3$ in der 3-, 4- und 5-Stellung des Benzolringes. Unter den Verbindungen der Formel I sind dabei diejenigen bevorzugt, in denen $R^4$ ein Niederalkylrest mit 1 bis 4 C-Atomen oder der Benzylrest ist.

Unter den genannten Verbindungen der allgemeinen Formel I sind weiter bevorzugt diejenigen, in denen $R^1$, $R^2$ und $R^3$ Wasserstoff, Methyl oder Methoxy bedeuten, und davon besonders bevorzugt solche Substanzen, in denen sich $R^1$, $R^2$ und $R^3$ in den Stellungen 3, 4 und 5 des Benzylrestes befinden und Methoxygruppen sind.

Die Verbindungen der allgemeinen Formel I sind antimikrobiell wirksam bei durch Bakterien und Protozoen hervorgerufenen Krankheiten und potenzieren, kombiniert mit Sulfonamiden, deren antibakterielle Wirkung. Sie können daher beispielsweise bei bakteriellen Erkrankungen der Atmungsorgane, Verdauungsorgane und Harnwege sowie bei Hals-, Nasen-, Ohreninfektionen und allgemein bei systemischen Infektionskrankheiten und bei Malaria verwendet werden.

Solche Sulfonamide sind beispielsweise:

2-Sulfanilamido-pyridin, 2-Sulfanilamido-thiazol, 2-Sulfanilamido-pyrimidin, 2-Sulfanilamido-4-methyl-pyrimidin, 2-Sulfanilamido-4,6-dimethyl-pyrimidin, 4-Sulfanilamido-2,6-dimethyl-pyrimidin, 5-Sulfanilamido-3,4-dimethyl-isoxazol, 3-Sulfanilamido-6-methoxy-pyridazin, 3-Sulfanilamido-6-chlor-pyridazin, 4-Sulfanilamido-2,6-dimethoxy-pyrimidin, 3-Sulfanilamido-2-phenyl-pyrazol, 2-Sulfanilamido-5-methyl-pyrimidin, 2-Sulfanilamido-5-methoxy-pyrimidin, 2-Sulfanilamido-5-methyl-isoxazol, 2-Sulfanilamido-4,5-dimethyl-oxazol, 2-Sulfanilamido-3-methoxy-pyrazin, 4-Sulfanilamido-5,6-dimethoxy-pyrimidin, 4-Sulfanilamido-3-methoxy-1,2,5-thiadiazol, 4-Aminobenzol-sulfonyl-guanidin.

Die Verbindungen der allgemeinen Formel I und ihre Salze können mit den beispielhaft genannten Sulfonamiden in verschiedenen Mischungsverhältnissen kombiniert werden, wobei das Verhältnis Verbindung allgemeine Formel I zu Sulfonamid von 1:10 bis 5:1 variieren kann. Bevorzugte Mischungsverhältnisse sind jedoch 1:1 bis 1:5. Dabei kommen in der Regel als Dosierung 20 bis 550 mg eines Wirkstoffes der allgemeinen Formel I in Betracht.

Die Herstellung der erfindungsgemäßen Verbindungen der allgemeinen Formel I geschieht nach den für die Herstellung von Amidinen üblichen Methoden, wie sie u.a. im Houben-Weyl "Methoden der organischen Chemie", Band 11/2 beschrieben sind, indem man einen Imidsäureester der allgemeinen Formel II

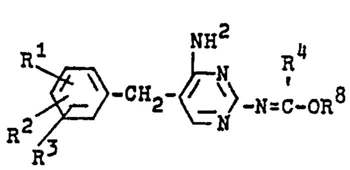

(II)

worin $R^1$, $R^2$, $R^3$ und $R^4$ die oben angegebene Bedeutung haben und $R^8$ niederes Alkyl mit 1—4 C-Atomen oder Benzyl bedeutet, mit einem Amin der allgemeinen Formel III

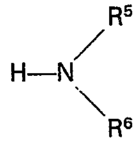

(III)

in welcher $R^5$ und $R^6$ die oben angegebene Bedeutung haben, zum Amidin der allgemeinen Formel I

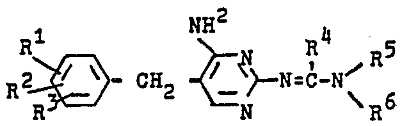

(I)

umsetzt und anschließend gegebenenfalls die so erhaltene Verbindung in ein pharmakologisch vertragliches Säureadditionssalz mit einer hierfür üblichen Säure überführt.

Die Imidsäureester der allgemeinen Formel II und ihre Herstellung sind in der deutschen Patentanmeldung P 27 30 468.3 vom 6. Juli 1977 der Anmelderin beschrieben.

Amine der allgemeinen Formel III können beispielsweise sein:

Ammoniak, Methylamin, Dimethylamin, Diäthylamin, Benzylamin, 3,4,5-Trimethoxybenzylamin, N-Phenyl-piperazin, Furfurylamin, Cyclohexylamin, Pyrrolidin, Piperidin, Morpholin, Anilin und Sulfonamide wie beispielsweise 2-Sulfanilamino-pyrimidin, 2-Sulfanilamido-4-methyl-pyrimidin, 2-Sulfanilamido-5-methyl-pyrimidin, 2-Sulfanilamido-5-isopropyl-pyrimidin, 2-Sulfanilamido-5-methoxy-pyrimidin, 3-Sulfanilamido-6-methoxy-pyridazin, 2-Sulfanilamido-3-methoxy-pyrazin, 3-Sulfanilamido-5-methyl-isoxazol und 2-Sulfanilamido-pyridin.

Die Herstellung dieser Verbindungen kann mit oder ohne Lösungsmittel erfolgen. Als mögliche Lösungsmittel eignen sich beispielsweise Pyridin, Athanol oder Wasser bzw. Gemische dieser Lösungsmittel. Die Reaktionstemperaturen liegen zwischen 0 und 150°C, bevorzugt zwischen 20 und 120°C bzw. bei Temperaturen bis zum Siedepunkt des verwendeten Lösungsmittels.

Verwendet man beispielsweise N-[4-Amino-5-(3,4,5-trimethoxybenzyl)-pyrimidin-2-yl]-acetamidsäureäthylester und Anilin als Ausgangsstoffe, so kann der Reaktionsverlauf durch folgendes Formelschema wiedergegeben werden:

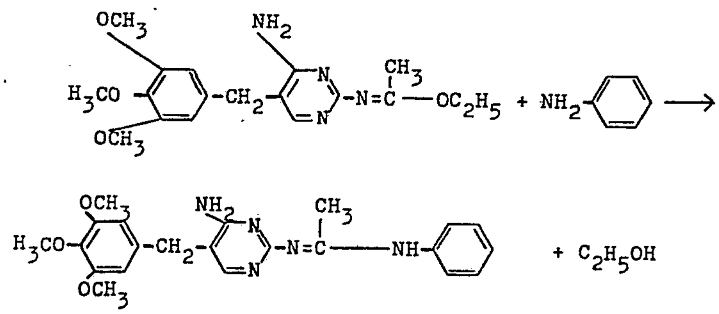

Zum Wirksamkeitsnachweis wurden erfindungsgemäße Substanzen im Tierversuch am Modell der sogenannten Aronson-Sepsis, wobei mit Streptococcus agalactiae infiziert wird, geprüft und mit dem bekannten Trimethoprim verglichen. Hierzu wurden Gruppen von je 30 weiblichen Mäusen mit einer tödlichen Dosis von Streptococcus agalactiae 7941 infiziert und 2 Stunden nach der Infektion mit einer Mischung von 300 mg 2-Sulfanilamido-4,5-dimethyloxazol + 60 mg einer der erfindungsgemäßen Substanzen behandelt. Außer einer nicht behandelten Kontrollgruppe wurde eine zweite Gruppe mit dem als Referenzsubstanz dienenden Gemisch von 300 mg 2-Sulfanilamido-4,5-dimethyloxazol + 60 mg Trimethoprim behandelt. Nach 44 Stunden wurde die Zahl der überlebenden Tiere bestimmt und diese Zahl durch die Zahl der überlebenden aus der mit der Referenzsubstanz behandelten Gruppe dividiert. Der so erhaltene Zahlenwert (Trimethoprimfaktor) ist ein Maß für die Wirkung der erfindungsgemäßen Substanzen im Vergleich zum Trimethoprim. F = 2 bedeutet also, daß die Substanz doppelt so wirksam ist wie Trimethoprim. Aus der folgenden Tabelle geht eine Überlegenheit der erfindungsgemäßen Substanzen gegenüber dem Trimethoprim bis zum 5,4-fachen hervor.

TABELLE
Allgemeine Formel

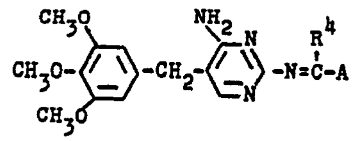

| Nr. | $R^4$ | A | F |
|---|---|---|---|
| 1 | $CH_3$— | 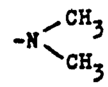 | 1,2 |
| 2 | $CH_3$— | 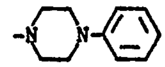 | 1,7 |
| 3 | $CH_3$— | 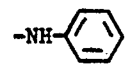 | 1,2 |
| 4 | $CH_3$— | 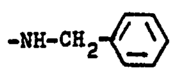 | 1,3 |
| 5 | $CH_3$— | 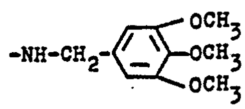 | 1,0 |
| 6 | $CH_3$— | 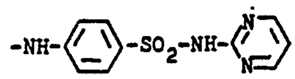 | 5,4 |
| 7 | $CH_3$— | 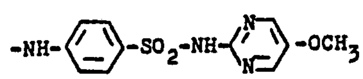 | 1,6 |
| 8 | $CH_3$— | 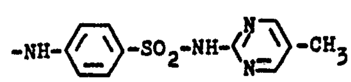 | 5,1 |
| 9 | $CH_3$— | 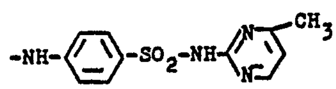 | 1,25 |
| 10 | $CH_3$— | 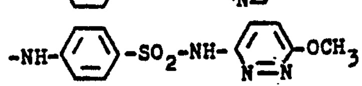 | 1,0 |

Gegenstand der Erfindung sind demnach auch chemotherapeutische Mittel, die neben üblichen Träger- und Verdünnungsmitteln eine Verbindung der allgemeinen Formel I, insbesondere in Kombination mit einem Sulfonamid als Wirkstoffe enthalten, sowie die Verwendung der Verbindungen der allgemeinen Formel I als Sulfonamidpotentiatoren.

Die chemotherapeutischen Mittel bzw. Zubereitungen werden mit den üblichen Trägerstoffen oder Verdünnungsmitteln und den üblicherweise verwendeten pharmazeutisch-technischen Hilfsstoffen entsprechend der gewünschten Applikationsart in bekannter Weise hergestellt.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen.

Beispiel 1

16,2 g N - [4 - Amino - 5 - (3,4,5 - trimethoxybenzyl) - pyrimidin - 2 - yl] - acetimidsäure - äthylester werden in 120 ml Pyridin gelöst und mit 14 ml gesättigter äthanolischer Ammoniaklösung versetzt und 12 Stunden bei 70°C gerührt. Nach Einengen im Vacuum wird der Rückstand aus Dioxan umkristallisiert. Man erhält 8,6 g (57,7% d.Th.) N - [4 - Amino - 5 - (3,4,5 - trimethoxybenzyl) - pyrimidin - 2 - yl] - acetamidin mit dem Fp.: 207°C.

Beispiel 2

1,8 g N - [4 - Amino - 5 - (3,4,5 - trimethoxybenzyl) - pyrimidin - 2 - yl] - acetimidsäure - äthylester werden mit 0,93 g Anilin und 20 ml absolutem Athanol unter Rühren 15 Stunden unter

Rückfluß zum Sieden erhitzt, abgekühlt und mit 80 ml Wasser versetzt. Man erhält 1,1 g (53% d.Th.) N - [4 - Amino - 5 - (3,4,5 - trimethoxybenzyl) - pyrimidin - 2 - yl] - N' - phenyl - acetamidin mit dem Fp.: 189°C.

Beispiel 3

1,8 g N - [4 - Amino - 5 - (3,4,5 - trimethoxybenzyl) - pyrimidin - 2 - yl] - acetimidsäure - äthylester werden mit 20 ml 40-prozentiger wässriger Dimethylaminlösung 10 Stunden bei 50—60°C gerührt. Der kristalline Niederschlag wird filtriert, mit Wasser gewaschen und aus Dioxan umkristallisiert. Es werden 1,24 g (69% d.Th.) N - [4 - Amino - 5 - (3,4,5 - trimethoxybenzyl) - pyrimidin - 2 - yl] - N',N' - dimethyl - acetamidin mit dem Fp.: 220°C erhalten.

Beispiel 4

Analog Beispiel 3 wurde N - [4 - Amino - 5 - (3,4,5 - trimethoxybenzyl) - pyrimidin - 2 - yl] - N',N' - diäthyl - acetamidin mit dem Fp.: 154°C aus N - [4 - Amino - 5 - (3,4,5 - trimethoxybenzyl) - pyrimidin - 2 - yl] - acetamidsäure - äthylester und Diäthylamin hergestellt.

Beispiel 5

1,8 g N - [(4 - Amino - 5 - (3,4,5 - trimethoxybenzyl) - pyrimidin - 2 - yl] - acetimidsäure - äthylester werden in 10 ml N-Phenylpiperazin suspendiert und auf 65°C erwärmt, bis alles gelöst ist, und danach 3 Stunden bei 90—100°C gerührt. Nach Abkühlung wird mit 100 ml Diäthyläther behandelt, der weißkristalline Niederschlag abfiltriert und aus Butylacetat umkristallisiert. Man erhält 1,6 g (67% d.Th.) N - [(4 - Amino - 5 - (3,4,5 - trimethoxybenzyl) - pyrimidin - 2 - yl] - N',N' - (3 - aza - 3 - phenyl - pentamethylen) - acetamidin mit dem Fp.: 197°C.

Beispiel 6

Analog Beispiel 5 wurde N - [4 - Amino - 5 - (3,4,5 - trimethoxybenzyl) - pyrimidin - 2 - yl] - N',N' - tetramethylen - acetamidin mit dem Fp.: 198°C aus N - [4 - Amino - 5 - (3,4,5 - trimethoxybenzyl) - pyrimidin - 2 - yl] - acetimidsäure - äthylester und Pyrrolidin hergestellt.

Beispiel 7

5,4 g N - [4 - Amino - 5 - (3,4,5 - trimethoxybenzyl) - pyrimidin - 2 - yl] - acetimidsäure - äthylester und 1,6 g Benzylamin werden in 40 ml Pyridin gelöst und 2 Stunden bei 90°C gerührt. Nach Einengen im Vakuum löst man den Rückstand in der Wärme in 200 ml Essigester, klärt mit Aktivkohle und kühlt ab. Aus der Lösung kristallisieren 3,2 g (51% d.Th.) N - [4 - Amino - 5 - (3,4,5 - trimethoxybenzyl) - pyrimidin - 2 - yl] - N' - benzyl - acetamidin mit dem Fp.: 164°C aus.

Analog Beispiel 7 wurden hergestellt:

8. N - [4 - Amino - 5 - (3,4,5 - trimethoxybenzyl) - pyrimidin - 2 - yl] - N' - (3,4,5 - trimethoxybenzyl) - acetamidin mit dem Fp.: 143°C aus N - [4 - Amino - 5 - (3,4,5 - trimethoxybenzyl) - pyrimidin - 2 - yl] - acetimidsäure - äthylester und 3,4,5 - Trimethoxybenzylamin.

9. N - [4 - Amino - 5 - (3,4,5 - trimethoxybenzyl) - pyrimidin - 2 - yl] - N',N' - (3 - oxa - pentamethylen) - acetamidin mit dem Fp.: 205°C aus N - [4 - Amino - 5 - (3,4,5 - trimethoxybenzyl) - pyrimidin - 2 - yl] - acetimidsäure - äthylester und Morpholin.

10. N - [4 - Amino - 5 - (3,4,5 - trimethoxybenzyl) - pyrimidin - 2 - yl] - N' - furfuryl - acetamidin mit dem Fp.: 184°C aus N - [4 - Amino - 5 - (3,4,5 - trimethoxybenzyl) - pyrimidin - 2 - yl] - acetimidsäure - äthylester und Furfurylamin.

11. N - [4 - Amino - 5 - (3,4,5 - trimethoxybenzyl) - pyrimidin - 2 - yl] - N' - cyclohexyl - acetamidin mit dem Fp.: 183°C aus N - [4 - Amino - 5 - (3,4,5 - trimethoxybenzyl) - pyrimidin - 2 - yl] - acetimidsäure - äthylester und Cyclohexylamin.

12. N - [4 - Amino - 5 - (3,4,5 - trimethoxybenzyl) - pyrimidin - 2 - yl] - N' - adamantylacetamidin mit dem Fp.: 248°C aus N - [4 - Amino - 5 - (3,4,5 - trimethoxybenzyl) - pyrimidin - 2 - yl] - acetimidsäure - äthylester und 1 - Adamantylamin.

Beispiel 13

10,8 g N - [4 - Amino - 5 - (3,4,5 - trimethoxybenzyl) - pyrimidin - 2 - yl] - acetamidsäure - äthylester werden mit 4,2 g 2 - Sulfanilamido - 5 - methoxy - pyrimidin (Sulfamethoxydiazin) in 80 ml Pyridin gelöst und 12 Stunden unter Rückfluß gerührt und das Reaktionsgemisch sodann im Vakuum eingeengt. Nach Lösen des Rückstandes in warmen Aceton und Behandlung mit Aktivkohle kristallisieren 5,43 g (60,8% d.Th.) N - [4 - Amino - 5 - (3,4,5 - trimethoxybenzyl) -

pyrimidin - 2- yl] - N' - [4 - (5 - methoxypyrimidin - 2 - yl) - sulfonamido] - phenyl - acetamidin mit dem Fp.: 218°C.

Analog Beispiel 13 wurden hergestellt:

14. N - [4 - Amino - 5 - (3,4,5 - trimethoxybenzyl) - pyrimidin - 2 - yl] - N' - [4 - (5 - methyl-pyrimidin - 2 - yl) - sulfonamido] - phenyl - acetamidin mit dem Fp.: 192°C aus N - [4 - Amino - 5 - (3,4,5 - trimethoxybenzyl) - pyrimidin - 2 - yl] - acetimidsäure - äthylester und 2 - Sulfanilamido - 5 - methyl - pyrimidin (Sulfaperin).

15. N - [4 - Amino - 5 - (3,4,5 - trimethoxybenzyl) - pyrimidin - 2 - yl] - N' - [4 - (pyrimidin - 2 - yl) - sulfonamido] - phenyl - acetamidin mit dem Fp.: 178°C (Zers.) aus N - [4 - Amino - 5 - (3,4,5 - trimethoxybenzyl) - pyrimidin - 2 - yl] - acetimidsäure - äthylester und 2 - Sulfanilamido - pyrimidin (Sulfadiazin).

16. N - [4 - Amino - 5 - (3,4,5 - trimethoxybenzyl) - pyrimidin - 2 - yl] - N' - [4 - (4 - methyl - pyrimidin - 2 - yl) - sulfonamido] - phenyl - acetamidin mit dem Fp.: 162°C aus N - [4 - Amino - 5 - (3,4,5 - trimethoxybenzyl) - pyrimidin - 2 - yl] - acetimidsäure - äthylester und 2 - Sulfanilamido - 4 - methyl - pyrimidin (Sulfamerazin).

17. N - [4 - Amino - 5 - (3,4,5 - trimethoxybenzyl) - pyrimidin - 2 - yl] - N' - (4 - sulfonylguanidino) - phenyl - acetamidin mit dem Fp.: 246°C aus N - [4 - Amino - 5 - (3,4,5 - trimethoxybenzyl) - pyrimidin - 2 - yl] - acetimidsäure - äthylester und 4 - Aminobenzolsulfonylguanidin (Sulfaguanidin).

18. N - [4 - Amino - 5 - (3,4,5 - trimethoxybenzyl) - pyrimidin - 2 - yl] - N' - [4 - (5 - methyl - isoxazol - 3 - yl) - sulfonamido] - phenyl - acetamidin mit dem Fp.: 155°C aus N - [4 - Amino - 5 - (3,4,5 - trimethoxybenzyl) - pyrimidin - 2 - yl] - acetimidsäure - äthylester und 3 - Sulfanilamido - 5 - methyl - isoxazol (Sulfamethoxazol).

19. N - [4 - Amino - 5 - (3,4,5 - trimethoxybenzyl) - pyrimidin - 2 -yl] - N' - [4 - (6 - methoxy - pyridazin - 3 - yl) - sulfonamido] - phenyl - acetamidin mit dem Fp.: 168°C aus N - [4 - Amino - 5 - (3,4,5 - trimethoxybenzyl) - pyrimidin - 2 - yl] - acetimidsäure - äthylester und 3 - Sulfanilamido - 6 - methoxy - pyridazin (Sulfamethoxy - pyridazin).

20. N - [4 - Amino - 5 - (3,4,5 - trimethoxybenzyl) - pyrimidin - 2 - yl] - N' - [4 - (pyridin - 2 - yl) - sulfonamido] - phenyl - acetamidin mit dem Fp.: 237°C aus N - [4 - Amino - 5 - (3,4,5 - trimethoxybenzyl) - pyrimidin - 2 - yl] - acetimidsäure - äthylester und 2 - Sulfanilamido - pyridin (Sulfapyridin).

21. N - [4 - Amino - 5 - (3,4,5 - trimethoxybenzyl) - pyrimidin - 2 - yl] - N' - [4 - (5 - isopropyl - pyrimidin - 2 - yl) - sulfonamido] - phenyl - acetamidin mit dem Fp.: 201°C aus N - [4 - Amino - 5 - (3,4,5 - trimethoxybenzyl) - pyrimidin - 2 - yl] - acetimidsäure - äthylester und 2 - Sulfanilamido - 5 - isopropyl - pyrimidin.

22. N - [4 - Amino - 5 - (3,4,5 - trimethoxybenzyl) - pyrimidin - 2 - yl] - N' - [4 - (3 - methoxy - pyrazin - 2 - yl) - sulfonamido] - phenyl - acetamidin mit dem Fp.: 148°C (Zers.) aus N - [4 - Amino - 5 - (3,4,5 - trimethoxybenzyl) - pyrimidin - 2 - yl] - acetimidsäure - äthylester und 2 - Sulfanilamido - 3 - methoxypyrazin (Sulfamethoxypyrazin, Sulfalen).

Beispiele für pharmazeutische Zubereitung

23. 400 mg 2 - Sulfanilamido - 4,5 - dimethyloxazol
    80 mg N - [4 - Amino - 5 - (3,4,5 - trimethoxybenzyl) - pyrimidin - 2 - yl] - N' - benzyl - acetamidin (Beispiel 7)
    20 mg Maisstärke
    10 mg Gelatine
    8 mg Talkum
    2 mg Magnesiumstearat
    20 mg Primojel

Die Wirkstoffe werden mit Maisstärke gemischt und mit wäßriger Gelatinelösung granuliert. Das trockene Granulat wird gesiebt und mit den Zuschlägen vermischt. Aus dieser Mischung werden in üblicher Weise Tabletten gepreßt.

24. 160 mg 2 - Sulfanilamido - 5 - methoxy - pyrimidin
80 mg N - [4 - Amino - 5 - (3,4,5 - trimethoxybenzyl) - pyrimidin - 2 - yl] - N' - phenyl - acetamidin (Beispiel 2)

5 mg Gelatine
30 mg Maisstärke
4 mg Talkum
1 mg Magnesiumstearat

Die Wirkstoffe werden mit wäßriger Gelatinelösung granuliert und nach dem Trocknen mit Maisstärke, Talkum und Magnesiumstearat vermischt. Aus dieser Mischung werden in üblicher Weise Tabletten gepreßt.

25. 400 mg N - [4 - Amino - 5 - (3,4,5 - trimethoxybenzyl) - pyrimidin - 2 - yl] - N' - [4 - pyrimidin - 2 - yl) - sulfonamido] - phenyl - acetamidin (Beispiel 15)

20 mg Maisstärke
10 mg Gelatine
8 mg Talkum
2 mg Magnesiumstearat
20 mg Primojel

Der Wirkstoff wird mit Maisstärke gemischt und mit wäßriger Gelatinelösung granuliert. Das trockene Granulat wird gesiebt und mit den Zuschlägen vermischt. Aus dieser Mischung werden in üblicher Weise Tabletten gepreßt.

26. 250 mg N - [4 - Amino - 5 - (3,4,5 - trimethoxybenzyl) - pyrimidin - 2 - yl] - N' - [4 - (5 - methoxypyrimidin - 2 - yl) - sulfonamido] - phenyl - acetamidin (Beispiel 13)
5 mg Gelatine
30 mg Maisstärke
4 mg Talkum
1 mg Magnesiumstearat

Der Wirkstoff wird mit wäßriger Gelatinelösung granuliert und nach dem Trocknen mit Maisstärke, Talkum und Magnesiumstearat vermischt. Aus dieser Mischung werden in üblicher Weise Tabletten gepreßt.

27. 4,00 g 2 - Sulfanilamido - 5 - methoxy - pyrimidin
2,00 g N - [4 - Amino - 5 - (3,4,5 - trimethoxybenzyl) - pyrimidin - 2 - yl] - N',N' - dimethyl - acetamidin (Beispiel 3)
1,9 g Tylose C 30
30,0 g Zucker
10,0 g Glycerin
2,5 g Bentonit
0,06 g Aroma
0,04 g Nipagin M
0,06 g Nipasol - Natrium
ad 100,00 g demineralisiertes Wasser

Die feinst gemahlenen Wirkstoffe werden in dem wäßrigen Tylose-Schleim suspendiert. Anschließend werden alle anderen Bestandteile unter Rühren nacheinander zugegeben. Zum Schluß wird mit Wasser auf 100,0 aufgefüllt.

28. 0,300 g N - [4 - Amino - 5 - (3,4,5 - trimethoxybenzyl) - pyrimidin - 2 - yl] - N' - [4 - (5 - methyl - pyrimidin - 2 - yl) - sulfonamido] - phenyl - acetamidin (Beispiel 14)
1,9 g Tylose C 30
30,0 g Zucker
10,0 g Glycerin
2,5 g Bentonit
0,06 g Aroma
0,04 g Nipagin M
0,06 g Nipasol - Natrium
ad 100,00 g demineralisiertes Wasser

Der feinst gemahlene Wirkstoff wird in dem wäßrigen Tylose-Schleim suspendiert. Anschließend werden alle anderen Bestandteile unter Rühren nacheinander zugegeben. Zum Schluß wird mit Wasser auf 100,0 aufgefüllt.

**Patentansprüche**

1. Amidino-benzylpyrimidine der allgemeinen Formel I

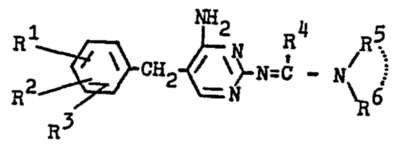 (I)

in der $R^1$, $R^2$ und $R^3$, die gleich oder verschieden voneinander sein können, Wasserstoff, Methyl, Methoxy oder Chlor bedeuten, $R^4$ Alkyl mit 1 bis 6 C-Atomen oder Benzyl bedeutet und $R^5$ und $R^6$, die gleich oder verschieden voneinander sein können, Wasserstoff, niederes Alkyl mit 1 bis 4 C-Atomen, Trimethoxybenzyl, Benzyl oder Phenyl, Cyclohexyl, Adamantyl oder Furfuryl oder einer der Reste $R^5$ oder $R^6$ die Gruppierung $—C_6H_4—SO_2—NH—R^7$ bedeutet, wobei $R^7$ den Rest

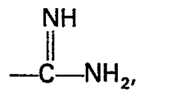, 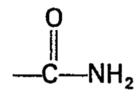

oder den Pyrimidin-2-yl-, 4-Methyl-pyrimidin-2-yl, 5-Methyl-pyrimidin-2-yl, 5-Isopropyl-pyrimidin-2-yl-, 5-Methoxy-pyrimidin-2-yl, 6-Methoxy-pyridazin-3-yl, 3-Methoxy-pyrazin-2-yl-, 5-Methyl-isoxazol-3-yl- oder 2-Pyridinyl-Rest bedeutet, oder in der $R^5$ und $R^6$ gemeinsam mit dem Stickstoff, mit dem sie verbunden sind, einen gesättigten heterocyclischen Ring mit 5 bis 7 Gliedern bilden, der gegenbenenfalls ein Sauerstoffatom oder die N—Y—Gruppe enthalten kann, wobei Y für Methyl, Benzyl oder Phenyl steht, sowie deren pharmakologisch verträglichen Salze mit hierfür üblichen Säuren.

2. N - [4 - Amino - 5 - (3,4,5 - trimethoxybenzyl) - pyrimidin - 2 - yl] - N',N' - (3 - aza - 3 - phenyl - pentamethylen) - acetamidin sowie seine pharmakologisch verträglichen Salze mit Säuren.

3. N - [4 - Amino - 5 - (3,4,5 - trimethoxybenzyl) - pyrimidin - 2 - yl] - N' - [4 - (5 - methoxypyrimidin - 2 - yl) - sulfonamido] - phenyl - acetamidin sowie seine pharmakologisch verträglichen Salze mit Säuren.

4. N - [4 - Amino - 5 - (3,4,5 - trimethoxybenzyl) - pyrimidin - 2 - yl] - N' - [4 - (5 - methyl - pyrimidin - 2 - yl) - sulfonamido] - phenyl - acetamidin sowie seine pharmakologisch verträglichen Salze mit Saüren.

5. N - [4 - Amino - 5 - (3,4,5 - trimethoxybenzyl) - pyrimidin - 2 - yl] - N' - [4 - pyrimidin - 2 - yl) - sulfonamido] - phenyl - acetamidin sowie seine pharmakologisch verträglichen Salze mit Säuren.

6. Verfahren zur Herstellung der Verbindungen gemäß Ansprüchen 1 bis 5, *dadurch gekennzeichnet,* daß man eine Verbindung der allgemeinen Formel II

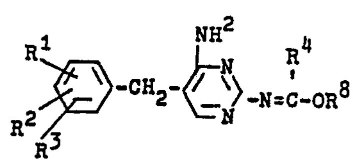 (II)

worin $R^1$, $R^2$, $R^3$ und $R^4$ die gleiche Bedeutung wie in der allgemeinen Formel I haben und $R^8$ niederes Alkyl mit 1 bis 4 C-Atomen oder Benzyl bedeutet, mit einem Amin der allgemeinen Formel III

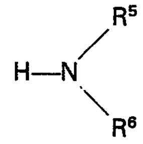 (III)

in welcher $R^5$ und $R^6$ die gleiche Bedeutung wie in der allgemeinen Formel I haben, gegebenenfalls in Gegenwart eines Lösungsmittels umsetzt, und, falls erwünscht, die so erhaltene Verbindung in ein pharmakologisch verträgliches Salz mit einer hierfür üblichen Säure überführt.

7. Arzneimittel, enthaltend eine Verbindung gemäß Ansprüchen 1 bis 5, gegebenenfalls zusammen mit einem antibakteriell wirksamen Sulfonamid, und nicht-toxischen, therapeutisch verträglichen festen oder flüssigen Trägerstoffen und galenischen Hilfsmitteln.

**Claims**

1. Amidino-benzylpyrimidines of the general formula I

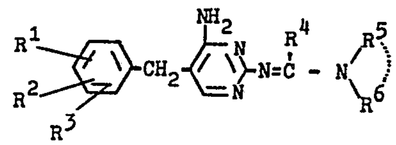

(I)

where $R^1$, $R^2$, and $R^3$, which may be identical or different, are hydrogen, methyl, methoxy or chlorine, $R^4$ is alkyl of 1 to 6 carbon atoms or benzyl and $R^5$ and $R^6$, which may be identical or different, are hydrogen, lower alkyl of 1 to 4 carbon atoms, trimethoxybenzyl, benzyl or phenyl, cyclohexyl, adamantyl or furfuryl, or one of the radicals $R^5$ and $R^6$ is $—C_6H_4—SO_2—NH—R^7$, where $R^7$ is

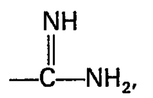

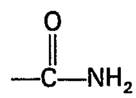

or pyrimidin-2-yl, 4-methyl-pyrimidin-2-yl, 5-methyl-pyrimidin-2-yl, 5-isopropyl-pyrimidin-2-yl, 5-methoxy-pyrimidin-2-yl, 6-methoxy-pyridazin-3-yl, 3-methoxy-pyrazin-2-yl, 5-methyl-isoxazol-3-yl or 2-pyridinyl, or $R^5$ and $R^6$ together with the nitrogen to which they are bonded are a saturated heterocyclic ring of 5—7 members, which may contain an oxygen atom or the N—Y group, where Y is methyl, benzyl or phenyl, and their pharmacologically acceptable salts with acids conventionally used for this purpose.

2. N - [4 - Amino - 5 - (3,4,5 - trimethoxybenzyl) - pyrimidin - 2 - yl] - N',N' - (3 - aza - 3 - phenyl - pentamethylene) - acetamidine and its pharmacologically acceptable salts with acids.

3. N - [4 - Amino - 5 - (3,4,5 - trimethoxybenzyl) - pyrimidin - 2 - yl] - N' - [4 - (5 - methoxypyrimidin - 2 - yl) - sulfonamido] - phenyl - acetamidine and its pharmacologically acceptable salts with acids.

4. N - [4 - Amino - 5 - (3,4,5 - trimethoxybenzyl) - pyrimidin - 2 - yl] - N' - 4 - (5 - methyl - pyrimidin - 2 - yl) - sulfonamido] - phenyl - acetamidine and its pharmacologically acceptable salts with acids.

5. N - [4 - Amino - 5 - (3,4,5 - trimethoxybenzyl) - pyrimidin - 2 - yl] - N' - [4 - (pyrimidin - 2 - yl) - sulfonamido] - phenyl - acetamidine and its pharmacologically acceptable salts with acids.

6. A process for the preparation of the compounds as claimed in claims 1 to 5, *characterized in that* a compound of the general formula II

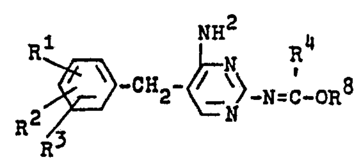

(II)

wherein $R^1$, $R^2$, $R^3$ and $R^4$ have the same meanings as in the general formula I and $R^8$ is lower alkyl of 1 to 4 carbon atoms or benzyl, is reacted with an amine of the general formula III

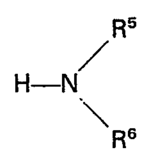

(III)

where $R^5$ and $R^6$ have the same meanings as in the general formula I, in the presence or absence of a solvent, after which the resulting compound may or may not be converted into a pharmacologically acceptable salt with an acid conventionally used for this purpose.

7. A drug containing a compound as claimed in claims 1 to 5, with or without an antibacterially active sulfonamide and non-toxic, therapeutically acceptable solid or liquid carriers and galenical assistants.

**Revendications**

1. Amidino-benzylpyrimidines de formule générale I

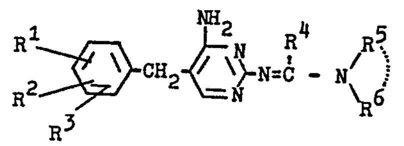

(I)

dans laquelle

$R^1$, $R^2$ et $R^3$, qui peuvent être identiques ou différents, représentent de l'hydrogène, un groupe méthyle ou méthoxy ou du chlore, $R^4$ est un reste alkyle en $C_1$ à $C_6$ ou un reste benzyle et $R^5$ et $R^6$, qui peuvent être identiques ou différents, représentent de l'hydrogène, un reste alkyle inferieur en $C_1$ à $C_4$, un reste triméthoxybenzyle, benzyle ou phényle, un reste cyclohexyle, adamantyle ou furfuryle, ou l'un des restes $R^5$ et $R^6$ représente le groupement $—C_6H_4—SO_2—NH—R^7$ dans lequel $R^7$ désigne le reste

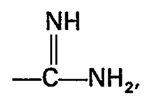

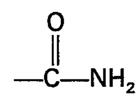

ou le reste pyrimidine-2-yle, 4-méthyl-pyrimidine-2-yle, 5-méthyl-pyrimidine-2-yle, 5-isopropyl-pyrimidine-2-yle, 5-méthoxy-pyrimidine-2-yle, 6-méthoxy-pyridazine-3-yle, 3-méthoxy-pyrazine-2-yle, 5-méthyl-isoxazol-3-yle ou 2-pyridinyle, ou $R^5$ et $R^6$ forment, simultanément avec l'azote sur lequel ils sont fixés, un hétérocycle saturé de 5 à 7 chaînons pouvant contenir, le cas échéant, un atome d'oxygène ou le groupe N—Y dans lequel Y représente un reste méthyle, benzyle ou phényle,

ainsi que leurs sels pharmacologiquement sans inconvénients pour l'individu, obtenus par réaction avec des acides couramment utilisés à cette fin.

2. N - [4 - Amino - 5 - (3,4,5 - triméthoxybenzyl) - pyrimidine - 2 - yl] - N',N' - (3 - aza - 3 - phényl - pentaméthylène) - acétamidine, ainsi que ses sels pharmacologiquement sans inconvénients pour l'individu, obtenus par réaction avec des acides couramment utilisés à cette fin.

3. N - [4 - Amino - 5 - (3,4,5 - triméthoxybenzyl) - pyrimidine - 2 - yl] - N' - [4 - (5 - méthoxypyrimidine - 2 - yl) - sulfamido] - phényl - acétamidine, ainsi que ses sels pharmacologiquement sans inconvénients pour l'individu, obtenus par réaction avec des acides couramment utilisés à cette fin.

4. N - [4 - Amino - 5 - (3,4,5 - triméthoxybenzyl) - pyrimidine - 2 - yl] - N' - [4 - (5 - méthyl - pyrimidine - 2 - yl) - sulfamido] - phényl - acétamidine, ainsi que ses sels pharmacologiquement sans inconvénients pour l'individu, obtenus par réaction avec des acides couramment utilisés à cette fin.

5. N - [4 - Amino - 5 - (3,4,5 - triméthoxybenzyl) - pyrimidine - 2 - yl] - N' - [4 - pyrimidine - 2 - yl) - sulfamido] - phényl - acétamidine, ainsi que ses sels pharmacologiquement sans inconvénients pour l'individu, obtenus par réaction avec des acides couramment utilisés à cette fin.

6. Procédé de préparation des composés selon l'une des revendications 1 à 5, caractérisé par le fait qu'on fait réagir, le cas échéant en présence d'un solvant, un composé de formule générale II

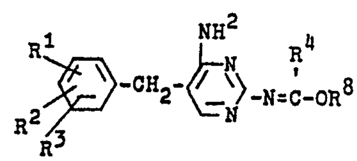

(II)

dans laquelle $R^1$, $R^2$, $R^3$ et $R^4$ ont la même signification que dans la formule générale I, $R^8$ représentant un reste alkyle inférieur en $C_1$ à $C_4$ ou un reste benzyle, avec une amine de formule générale III

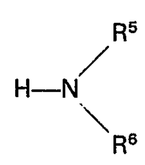

(III)

dans laquelle $R^5$ et $R^6$ ont la même signification que dans la formule générale I et, si désiré, on transforme le composé ainsi obtenu en un sel pharmacologiquement sans inconvénients pour l'individu, obtenu par réaction avec des acides couramment utilisés à cette fin.

7. Produits pharmaceutiques contenant un composé selon les revendications 1 à 5, le cas échéant en association avec un sulfamide à effet anti-bactérien, et des supports solides ou liquides non toxiques, thérapeutiquement sans inconvénients pour lesdits produits et l'individu, ainsi que des produits auxiliaires galéniques.